# EUROPEAN PATENT APPLICATION

(11) **EP 3 950 923 A1**
(43) Date of publication of application: **09.02.2022**
(21) Application number: 20779604.6
(22) Date of filing: 25.03.2020
(51) Int. Cl.: C12M 3/00, C12N 5/071

(54) **BRAIN BLOOD VESSEL MODEL AND DEVICE**

(30) Priority: 27.03.2019 JP 2019061326
(71) Applicant: Osaka University, Suita-shi, Osaka 565-0871 (JP)
(72) Inventor: MATSUSAKI Michiya, Suita-shi, Osaka 565-0871 (JP); FIGAROL Agathe Elisabeth Marie, Suita-shi, Osaka 565-0871 (JP); SATO Kaoru, Kawasaki-city, Kanagawa 210-9501 (JP)
(74) Representative: TBK
(86) International application number: PCT/JP2020/013477
(87) International publication number: WO 2020/196678

(57) **Abstract**

Disclosed is a brain blood vessel model composed of a three-dimensional tissue containing defibrated extracellular matrix components and cells including brain microvascular endothelial cells, pericytes, and astrocytes, wherein at least a portion of the above-described cells adheres to the above-described defibrated extracellular matrix.

## Description

### Technical Field

The present invention relates to a brain blood vessel model and a device.

### Background Art

The blood-brain barrier (BBB) is a barrier mechanism that limits exchange of substances between blood and the central nervous system. Brain blood vessels are complex tubular forms composed of pericytes (BPC) and brain microvascular endothelial cells (BMEC) backed with astrocytes (ASTR). Inflow of substances is limited to transportation by transporters due to such a strong barrier mechanism (BBB). In central nervous system drug discovery, it is necessary for candidate compounds to pass through the BBB to reach the brain. However, since there is no model for predicting intracerebral transferability in humans, this is a major cause of making the development of central nervous system drugs significantly difficult compared to other disease areas. Various models for predicting intracerebral transferability have been reported (for example, Non-Patent Literature 1).

### Citation List

### Non-Patent Literature

[Non-Patent Literature 1] Marco Campisi, et al., "3D self-organized microvascular model of the human blood-brain barrier with endothelial cells, pericytes and astrocytes" Biomaterials, 2018, 180, 117-129

### Summary of Invention

### Technical Problem

From the viewpoint of enabling good evaluation of extrapolation to humans in a non-clinical exploration stage, it is desirable to construct a three-dimensional tissue in which a brain blood vessel network is modeled in vitro.

The present invention has been made from the viewpoint of the above-described circumstances, and an object of the present invention is to provide a brain blood vessel model enabling good evaluation of extrapolation to humans. Another object of the present invention is to provide a device using the brain blood vessel model.

### Solution to Problem

The present inventors have conducted extensive studies, and as a result, they have found that the above-described problem can be solved by the method shown below.
[1] A brain blood vessel model composed of a three-dimensional tissue containing defibrated extracellular matrix components and cells including brain microvascular endothelial cells, pericytes, and astrocytes, wherein at least a portion of the cells adheres to the defibrated extracellular matrix components.
[2] The brain blood vessel model according to [1], wherein the defibrated extracellular matrix components are defibrated collagen components.
[3] The brain blood vessel model according to [1] or [2], wherein the three-dimensional tissue further contains fibrin.
[4] A method for producing a brain blood vessel model, including:
   a contacting step of bringing defibrated extracellular matrix components into contact with cells including brain microvascular endothelial cells, pericytes, and astrocytes in an aqueous medium;
   and a culture step of culturing the cells with which the defibrated extracellular matrix components are brought into contact.
[5] The method for producing a brain blood vessel model according to [4], in which the defibrated extracellular matrix components are defibrated collagen components.
[6] The method for producing a brain blood vessel model according to [4] or [5], in which the contacting step is performed in the presence of fibrin.
[7] A device including: a plate in which at least one well is provided; and a brain blood vessel model which is placed in the well and formed through self-organization.
[8] The device according to [7], wherein the brain blood vessel model is composed of a three-dimensional tissue construct containing defibrated extracellular matrix components and cells including brain microvascular endothelial cells, pericytes, and astrocytes, wherein at least a portion of the cells adheres to the defibrated extracellular matrix components.

### Advantageous Effects of Invention

According to the present invention, it is possible to provide a brain blood vessel model enabling good evaluation of extrapolation to humans. The brain blood vessel model of the present invention includes human BMEC, BPC, and ASTR, has a tube structure and a high throughput, and enables perfusion culture, in which expression of a transporter is confirmed. According to the present invention, it is possible to provide a device using the brain blood vessel model.

### Brief Description of Drawings

Fig. 1 shows micrographs illustrating observation results of a brain blood vessel model produced using a human established cell line through CD31 staining.
Fig. 2 is a photograph illustrating a fluorescence observation result of a frozen section of a three-dimensional tissue through CD31 staining.
Fig. 3 is a view illustrating results of confirming expression of proteins in a brain blood vessel model through immunostaining.
Fig. 4 is a view illustrating results of confirming expression of proteins in a brain blood vessel model through western blotting.
Fig. 5 shows micrographs illustrating observation results of a brain blood vessel model produced using an iPS cell-derived iBMEC through CD31 staining.
Fig. 6 is a schematic diagram illustrating a brain blood vessel model having openings in its lower portion.
Fig. 7 shows micrographs illustrating observation results of a brain blood vessel model having openings in its lower portion through CD31 staining.
Fig. 8 is a photograph illustrating an observation result of a brain blood vessel model having openings in its lower portion.
Fig. 9 is a view illustrating results of confirming effects of a brain blood vessel model caused by adding FD-2000 kDa.
Fig. 10 is a view illustrating analysis results of defibrated collagen components.
Fig. 11 shows photographs illustrating fluorescence observation results of (b) a three-dimensional tissue produced by shear stress culture and (a) a three-dimensional tissue produced by static culture through CD31 staining.
Fig. 12 shows micrographs illustrating observation results of (b) a three-dimensional tissue produced by shear stress culture and (a) a three-dimensional tissue produced by static culture through DAB staining.
Fig. 13 is a graph illustrating results obtained by comparing expression levels between genes in a three-dimensional tissue produced by shear stress culture and a three-dimensional tissue produced by static culture.
Fig. 14 shows views illustrating results of confirming expression of proteins through fluorescent immunostaining in (b) a three-dimensional tissue produced by shear stress culture and (a) a three-dimensional tissue produced by static culture.

### Description of Embodiments

Hereinafter, embodiments of the present invention will be described in detail. However, the present invention is not limited to the following embodiments.

### <Brain Blood Vessel Model>

A brain blood vessel model according to one embodiment is composed of a three-dimensional tissue which contains defibrated extracellular matrix components and cells including brain microvascular endothelial cells, pericytes, and astrocytes and in which at least a portion of the cells adheres to the defibrated extracellular matrix components. Since the brain blood vessel model is an evaluation model in which a human blood-brain barrier in a state closer to a living body is reproduced, intracerebral transferability or the like of central nerve system drugs can be predicted with high accuracy.

The brain blood vessel model" is a tissue model which is constructed in vitro and contains at least brain microvascular endothelial cells, pericytes, and astrocytes and in which at least a part of a structure of brain blood vessels is reproduced. The brain blood vessel model has strong tight junctions (with a barrier function) with brain microvascular endothelial cells, pericytes, and astrocytes. The brain blood vessel model is an in vitro model of a blood-brain barrier (BBB).

### [Three-Dimensional Tissue]

The "three-dimensional tissue" means an aggregate of cells in which the cells are three-dimensionally arranged and which is artificially produced through cell culture. In the three-dimensional tissue, cells may be three-dimensionally arranged via a scaffold material such as an extracellular matrix. The shape of the three-dimensional tissue is not particularly limited, and examples thereof include a sheet shape, a spherical shape, an ellipsoidal shape, and a rectangular parallelepiped shape. Here, biological tissue has a more complicated configuration than the three-dimensional tissue. For this reason, it is possible to easily distinguish the three-dimensional tissue from biological tissue.

### (Cells)

In one embodiment, the three-dimensional tissue contains brain microvascular endothelial cells, pericytes, and astrocytes as cells. Examples of animal species from which cells are derived include humans, pigs, cattle, and mice. As the above-described cells, human established cell lines may be used, or iPS cell- or ES cell-derived cells may be used, for example. For example, cells available from RIKEN Cell Bank, Takara Bio Inc., and the like can be used The iPS cells used may have been manufactured in-house.

In the three-dimensional tissue, the cell number ratio between the brain microvascular endothelial cells, the pericytes, and the astrocytes (brain microvascular endothelial cells: pericytes: astrocytes) may be 99:0.5:0.5 to 0.5:49.75:49.75 or 50:25:25 to 25:37.5:37.5.

The three-dimensional tissue may contain one or more kinds of cells (other cells) in addition to the brain microvascular endothelial cells, the pericytes, and the astrocytes.

The content of cells based on the total mass of the three-dimensional tissue may be greater than or equal to 0.01 mass%, greater than or equal to 0.1 mass%, greater than or equal to 0.5 mass%, greater than or equal to 1 mass%, or greater than or equal to 25 mass% and may be less than or equal to 99 mass% or less than or equal to 75 mass%, for example. The content of cells based on the total mass of the three-dimensional tissue may be, for example, 0.01 to 99 mass%, 0.1 to 99 mass%, 1 to 99 mass%, or 25 mass% to 75 mass%.

### (Defibrated Extracellular Matrix Components)

In one embodiment, the three-dimensional tissue contains defibrated extracellular matrix components. When the three-dimensional tissue contains defibrated extracellular matrix components, a brain blood vessel model having a tube structure is more easily produced.

The extracellular matrix components are extracellular matrix molecule aggregates formed by a plurality of extracellular matrix molecules. Extracellular matrix molecules mean substances existing extracellularly in an organism. Any substances can be used as extracellular matrix molecules as long as these do not adversely affect growth of cells and formation of cell aggregates. Examples of extracellular matrix molecules include collagen, laminin, fibronectin, vitronectin, elastin, tenascin, entactin, fibrillin, and proteoglycan, but the present invention is not limited thereto. These extracellular matrix components may be used alone or in combination. Extracellular matrix molecules may be substances obtained by modifying the above-described extracellular matrix molecules or variants of the above-described extracellular matrix molecules as long as these do not adversely affect the growth of cells and the formation of cell aggregates.

Examples of collagen include fibrous collagen and non-fibrous collagen. Fibrous collagen means collagen that is a main component of collagen fibers, and specific examples thereof include type I collagen, type II collagen, and type III collagen. Examples of non-fibrous collagen include fibrous type IV collagen.

Examples of proteoglycans include, but are not limited to, chondroitin sulfate proteoglycans, heparan sulfate proteoglycans, keratan sulfate proteoglycans, and dermatan sulfate proteoglycans.

An extracellular matrix component may contain at least one selected from the group consisting of collagen, laminin, and fibronectin and preferably contains collagen. Collagen is preferably fibrous collagen and more preferably type I collagen. Commercially available collagen may be used as fibrous collagen, and specific examples thereof include a porcine skin-derived type I collagen freeze-dried substance manufactured by NH Foods Ltd.

An extracellular matrix component may be derived from animals. Examples of animal species from which extracellular matrix components are derived include, but are not limited to, humans, pigs, and cattle. A component derived from one type of animal may be used as an extracellular matrix component, or components derived from plural kinds of animals may be used in combination. The animal species from which extracellular matrix components are derived may be the same as or different from the origin of cells to be three-dimensionally constructed.

Defibrated extracellular matrix components are components obtained by defibrating the above-described extracellular matrix components through application of physical force. Defibration is performed under the conditions of not cleaving bonds in extracellular matrix molecules. The method for defibrating extracellular matrix components is not particularly limited. For example, extracellular matrix components may be defibrated through application of physical force with an ultrasonic homogenizer, a stirring homogenizer, a high-pressure homogenizer, and the like. In a case of using a stirring homogenizer, extracellular matrix components may be homogenized as they are or may be homogenized in an aqueous medium such as physiological saline. In addition, millimeter-sized and nanometer-sized defibrated extracellular matrix components can also be obtained by adjusting the time, the number of times of homogenizing or the like. Defibrated extracellular matrix components can also be obtained through defibration by repeating freezing and thawing.

Defibrated extracellular matrix components preferably include defibrated collagen components. In the defibrated collagen components, a triple helix structure derived from collagen is maintained.

Examples of the shape of defibrated extracellular matrix components include a fibrous shape. The fibrous shape means a shape composed of a filamentous extracellular matrix component or a shape composed of cross-linked filamentous extracellular matrix components.

The average length of defibrated extracellular matrix components may be greater than or equal to 1 nm, greater than or equal to 5 nm, or greater than or equal to 10 nm, and may be less than or equal to 1 mm, for example. In one embodiment, the average length of defibrated extracellular matrix components may be 10 nm to 1 mm and may be 22 µm to 400 µm or 100 µm to 400 µm from the viewpoint of facilitating formation of thick tissue, for example. In another embodiment, the average length of defibrated extracellular matrix components may be less than or equal to 100 µm, less than or equal to 50 µm, less than or equal to 30 µm, less than or equal to 15 µm, less than or equal to 10 µm, or less than or equal to 1 µm from the viewpoint of facilitating stable tissue formation. Of all the defibrated extracellular matrix components, the average length of most of the defibrated extracellular matrix components may be within the above-described numerical ranges. Specifically, of all the defibrated extracellular matrix components, the average length of 99% of the defibrated extracellular matrix components may be within the above-described numerical ranges. Defibrated extracellular matrix components are preferably defibrated collagen components having an average length within the above-described ranges.

The average diameter of defibrated extracellular matrix components may be 10 nm to 100 µm, 4 µm to 30 µm, or 20 µm to 30 µm. Defibrated extracellular matrix components may be defibrated collagen components having an average diameter within the above-described ranges.

The above-described ranges of the average length and the average diameter are optimized from the viewpoint of tissue formation. Therefore, it is desirable that the average length and the average diameter fall within the above-described ranges at a stage where defibrated extracellular matrix components are suspended in an aqueous medium to form tissue.

The average length and the average diameter of defibrated extracellular matrix components can be obtained by measuring each defibrated extracellular matrix component using an optical microscope and performing image analysis. In the present specification, the "average length" means an average value of the lengths of the measured samples in the longitudinal direction and the "average diameter" means an average value of the lengths of the measured samples in the direction orthogonal to the longitudinal direction.

The content of extracellular matrix in a three-dimensional tissue based on the three-dimensional tissue (dry weight) may be 0.01 to 99 mass%, 10 to 90 mass%, 10 to 80 mass%, 10 to 70 mass%, 10 to 60 mass%, 1 to 50 mass%, 10 to 50 mass%, 10 to 30 mass%, or 20 to 30 mass%. The content of extracellular matrix in a three-dimensional tissue means a total content of extracellular matrix constituting the three-dimensional tissue. The total content of extracellular matrix constituting a three-dimensional tissue is a total content of extracellular matrix (endogenous extracellular matrix) produced by cells constituting the three-dimensional tissue and exogenous extracellular matrix derived from the above-described defibrated extracellular matrix components or the like.

The content of extracellular matrix in a three-dimensional tissue can be calculated from the volume of an obtained three-dimensional tissue and the mass of a decellularized three-dimensional tissue, for example. In addition, the content of extracellular matrix in a three-dimensional tissue can be measured by, for example, a method such as ELISA in which an antigen-antibody reaction is used or a chemical detection method such as QuickZyme.

The content of collagen in a three-dimensional tissue may be within the above-described ranges. Examples of the method for measuring the content of collagen in a three-dimensional tissue include the following method for quantitatively determining hydroxyproline. Hydrochloric acid (HCl) is mixed with a solution in which a three-dimensional tissue is dissolved, and the mixed solution is incubated at a high temperature for a predetermined time. Then, the temperature is returned to room temperature, and a centrifuged supernatant is diluted to a predetermined concentration to prepare a sample. A hydroxyproline standard solution is treated in the same manner as the sample, and then diluted stepwise to prepare a standard Each of the sample and the standard is subjected to a predetermined treatment with hydroxyproline assay buffer and a detection reagent, and the absorbance at 570 nm is measured. The amount of collagen is calculated by comparing the absorbance of the samples with that of the standard. A solution obtained by directly suspending and dissolving a three-dimensional tissue in high-concentration hydrochloric acid may be centrifuged, and a supernatant may be collected to be used for quantitative determination of collagen. In addition, the three-dimensional tissue to be dissolved may be in a state where it has been collected from a culture liquid, or may be dissolved in a state where a liquid component has been removed by performing a drying treatment after collection. However, in the case where a three-dimensional tissue in a state where it has been collected from a culture liquid is dissolved to perform quantitative determination of collagen, it is expected that the measurement value of the weight of the three-dimensional tissue will vary due to the influence of medium components absorbed by the three-dimensional tissue and the remainder of a medium due to a problem of an experimental technique. Therefore, it is preferable to use the weight after drying as a reference from the viewpoint of stably measuring the amount of collagen making up the tissue per weight and unit weight.

More specific examples of the method for measuring the content of collagen include the following method.

### (Preparation of Sample)

The total amount of a freeze-dried three-dimensional tissue is mixed with 6 mol/L HCl and the mixture is incubated in a heat block at 95°C for 20 hours or longer, and then the temperature is returned to room temperature. After centrifugation at 13,000 g for 10 minutes, a supernatant of the sample solution is collected. After the supernatant is appropriately diluted with 6 mol/L HCl so that measurement results to be described below fall within a range of a calibration curve, 200 µL of the supernatant is diluted with 100 µL of ultrapure water to prepare a sample. 35 µL of the sample is used.

### (Preparation of Standard)

125 µL of a standard solution (1,200 µg/mL in acetic acid) and 125 µL of 12 mol/L HCl are placed in a screw-cap tube and mixed with each other, and the mixture is incubated in a heat block at 95°C for 20 hours. Then, the temperature is returned to room temperature. After centrifugation at 13,000 g for 10 minutes, a supernatant is diluted with ultrapure water to prepare 300 µg/mL S1, and the S1 is diluted stepwise with ultrapure water to prepare S2 (200 µg/mL), S3 (100 µg/mL), S4 (50 µg/mL), S5 (25 µg/mL), S6 (12.5 µg/mL), and S7 (6.25 µg/mL). S8 (0 µg/mL) containing only 90 µL of 4 mol/L HCl is also prepared.

### (Assay)

35 µL of each of the standard and the samples is placed on a plate (included in the QuickZyme Total Collagen Assay Kit, QuickZyme Biosciences). 75 µL of assay buffer (included in the above-described kit) is added to the wells. The plate is closed with a seal and incubated at room temperature while being shaken for 20 minutes. The seal is removed, and 75 µL of a detection reagent (reagent A: reagent B = 30 µL: 45 µL, included in the above-described kit) is added to the wells. The plate is closed with a seal, and the solutions are mixed with each other through shaking and incubated at 60°C for 60 minutes. The mixture is sufficiently cooled on ice, and the seal is removed to measure the absorbance at 570 nm. The amount of collagen is calculated by comparing the absorbance of the samples with that of the standard.

In addition, the collagen making up a three-dimensional tissue may be defined by an area ratio or a volume ratio thereof. The "definition by an area ratio or a volume ratio thereof' means that the proportion of a region where collagen making up the entire three-dimensional tissue is present is calculated through naked-eye observation or using various kinds of microscopes, image analysis software, and the like after the collagen in the three-dimensional tissue is made to be distinguishable from other tissue components through, for example, a well-known staining technique (for example, immunostaining in which an anti-collagen antibody is used or Masson's Trichrome staining). In a case where the definition is performed by an area ratio, any cross section or surface in a three-dimensional tissue may be used to define the area ratio without limitation.

For example, in a case where collagen in a three-dimensional tissue is defined by an area ratio, the proportion of the area thereof may be 0.01% to 99%, 1% to 99%, 5% to 90%, 7% to 90%, 20% to 90%, or 50% to 90% based on the total area of the above-described three-dimensional tissue. "Collagen in a three-dimensional tissue" is as described above. The proportion of the area of collagen constituting a three-dimensional tissue means a proportion of the total area of endogenous collagen and exogenous collagen. The above-described proportion of the area of collagen can be calculated, for example, as a proportion of an area of collagen stained blue with respect to the total cross-sectional area passing through a substantially central portion of an obtained three-dimensional tissue subjected to Masson's Trichrome staining.

### [Other Components]

In one embodiment, the three-dimensional tissue may contain other components in addition to the defibrated extracellular matrix components and the cells. Other components may be, for example, the above-described extracellular matrix components. Examples of other components include fibrin, laminin, chondroitin sulfate, type IV collagen, hyaluronic acid, fibronectin, tenascin, and matrigel. The other components may be at least one component selected from the group consisting of fibrin and laminin. Another component may be fibrin from the viewpoint of superior network formation.

In a case of using fibrin as another component, the content of fibrin in a three-dimensional tissue based on the total mass (dry mass) of the three-dimensional tissue may be, for example, 0.1 to 99 mass%, 1 to 50 mass%, 3 to 20 mass%, or 5 mass% to 10 mass%. The content of fibrin in a three-dimensional tissue can be measured through weight measurement, absorbance measurement, an ELISA method, or the like.

### [Shape of Three-Dimensional Tissue]

The thickness of a three-dimensional tissue may be greater than or equal to 10 µm, greater than or equal to 100 µm, or greater than or equal to 1,000 µm. Such a three-dimensional tissue is a structure closer to biological tissue, and is suitable as a substitute for a laboratory animal or the like. The upper limit of the thickness thereof is not particularly limited, but may be, for example, less than or equal to 10 mm, less than or equal to 3 mm, less than or equal to 2 mm, less than or equal to 1.5 mm, and less than or equal to 1 mm.

Here, the "thickness of a three-dimensional tissue" means the distance between both ends in a direction perpendicular to the main surface in a case where the three-dimensional tissue has a sheet shape or a rectangular parallelepiped shape. In a case where the above-described main surface is uneven, the thickness means the distance therebetween at the thinnest portion of the above-described main surface. In addition, in a case where a three-dimensional tissue has a spherical shape, the thickness thereof means the diameter thereof In addition, in a case where a three-dimensional tissue has an ellipsoidal shape, the thickness thereof means the minor axis thereof. In a case where a three-dimensional tissue has a substantially spherical shape or a substantially ellipsoidal shape and its surface is uneven, the thickness thereof means the shortest distance between two points where a straight line passing through the gravity center of the three-dimensional tissue and the above-described surface intersect.

A residual rate of a three-dimensional tissue subjected to trypsin treatment at a trypsin concentration of 0.25%, a temperature of 37°C, a pH of 7.4, and a reaction time of 15 minutes may be greater than or equal to 70%, greater than or equal to 80%, or greater than or equal to 90%. Such a three-dimensional tissue is stable because it is unlikely to be decomposed by an enzyme during or after culturing. The above-described residual rate can be calculated from the mass of a three-dimensional tissue before and after trypsin treatment, for example.

The residual rate of a three-dimensional tissue subjected to collagenase treatment at a collagenase concentration of 0.25%, a temperature of 37°C, a pH of 7.4, and a reaction time of 15 minutes may be greater than or equal to 70%, greater than or equal to 80%, or greater than or equal to 90%. Such a three-dimensional tissue is stable because it is unlikely to be decomposed by an enzyme during or after culturing.

### <Method for Producing Brain Blood Vessel Model>

A method for producing a brain blood vessel model according to one embodiment includes: a contacting step of bringing defibrated extracellular matrix components into contact with cells including brain microvascular endothelial cells, pericytes, and astrocytes in an aqueous medium; and a culture step of culturing the cells with which the defibrated extracellular matrix components are brought into contact.

The "aqueous medium" means a liquid having water as an essential component. The aqueous medium is not particularly limited as long as exogenous collagen and cells can be stably present Examples of thereof include physiological saline such as phosphate-buffered physiological saline (PBS) and liquid media such as a Dulbecco's Modified Eagle medium (DMEM) and a vascular endothelial cell-exclusive medium (EGM2). The liquid medium may be a mixed medium obtained by mixing two kinds of media with each other. The aqueous medium is preferably a liquid medium from the viewpoint of reducing a load on cells.

### [Contacting Step]

The method for bringing defibrated extracellular matrix components into contact with cells in an aqueous medium is not particularly limited. Examples thereof include a method for adding a dispersion of defibrated extracellular matrix components to a culture liquid containing cells, a method for adding cells to a medium dispersion of defibrated extracellular matrix components, or a method for adding defibrated extracellular matrix components and cells to each previously prepared aqueous medium.

Defibrated extracellular matrix components are components obtained by defibrating the above exemplified extracellular matrix components. Defibrated extracellular matrix components may include defibrated collagen components.

The concentration of defibrated extracellular matrix components in an aqueous medium in the contacting step can be appropriately determined depending on the shape and thickness of a target three-dimensional tissue, the size of an incubator, or the like. For example, the concentration of defibrated extracellular matrix components in an aqueous medium in the contacting step may be 0.1 to 90 mass% or may be 1 to 30 mass%.

The amount of defibrated extracellular matrix components used in the contacting step based on 1.0 × 10⁵ to 10.0 × 10⁵ cells (number of cells) may be greater than or equal to 0.1 mg, greater than or equal to 0.5 mg, greater than or equal to 1.0 mg, greater than or equal to 2.0 mg, or greater than or equal to 3.0 mg, and may be less than or equal to 100 mg or less than or equal to 50 mg, for example. Defibrated extracellular matrix components may be added to 2.0 × 10⁵ to 8.0 × 10⁵ cells, 3.0 × 10⁵ to 6.0 × 10⁵ cells, or 5 × 10⁵ cells so as to be in the above-described ranges.

The mass ratio of defibrated extracellular matrix components to cells in the contacting step (defibrated extracellular matrix components: cells) may be 1,000:1 to 1:1, 900:1 to 9:1, or 500:1 to 10:1.

The cell number ratio between brain microvascular endothelial cells, pericytes, and astrocytes (brain microvascular endothelial cells: pericytes: astrocytes) in the contacting step may be 99:0.5:0.5 to 0.5:49.75:49.75 or 50:25:25 to 25:37.5:37.5.

A step of precipitating defibrated extracellular matrix components and cells together in an aqueous medium (precipitation step) may be further provided between the contacting step and the culture step. By performing such a step, the distribution of the defibrated extracellular matrix components and the cells in a three-dimensional tissue becomes more uniform. The specific method is not particularly limited, but examples thereof include a method for centrifuging a culture liquid containing defibrated extracellular matrix components and cells.

The contacting step may be performed in the presence of other components in addition to the defibrated extracellular matrix components and the cells. Other components may be, for example, the above-described components. Another component may be fibrin from the viewpoint of superior network formation.

In a case where the contacting step is performed in the presence of fibrin, the contacting step may be, for example, a step of bringing defibrated extracellular matrix components into contact with cells in an aqueous solution containing fibrin and an aqueous medium. The amount of fibrin used in the contacting step can be appropriately set based on network formation. The content of fibrin in the contacting step when an aqueous solution containing fibrin and an aqueous medium is set to 100 mass% may be, for example, greater than or equal to 1 mass% or greater than or equal to 5 mass%, and may be, for example, less than or equal to 99 mass% or less than or equal to 90 mass%.

### [Culture Step]

The method for culturing cells to which defibrated extracellular matrix components are brought into contact is not particularly limited, and a suitable culture method can be performed depending on the types of cells to be cultured. For example, the culture temperature may be 20°C to 40°C or may be 30°C to 37°C. The pH of a medium may be 6 to 8 or may be 7.2 to 7.4. The culture time may be 1 day to 2 weeks or 1 week to 2 weeks.

The medium is not particularly limited, and a suitable medium can be selected depending on the types of cells to be cultured Examples of media include an Eagle's MEM medium, DMEM, a Modified Eagle medium (MEM), a Minimum Essential medium, RPMI, and a GlutaMax medium. The medium may be a medium to which serum is added, or may be a serum-free medium. The medium may be a mixed medium obtained by mixing two kinds of media with each other.

The cell density in a medium in the culture step can be appropriately determined depending on the shape and thickness of a target three-dimensional tissue, the size of an incubator, or the like. For example, the cell density in a medium in the culture step may be 1 to 10⁸ cells/mL, or may be 10³ to 10⁷ cells/mL. In addition, the cell density in a medium in the culture step may be the same as that in an aqueous medium in the contacting step.

The culture method is not particularly limited as long as the object of the present invention can be achieved, and may include, for example, static culture or shear stress culture. The shear stress culture includes culture performed by applying fluid shear stress to cells to be cultured by intentionally constantly moving a medium coming into contact with the cells. By applying fluid shear stress to the cells for culture, a three-dimensional tissue having a clearer brain blood vessel tube network structure compared to static culture can be produced. In addition, by applying fluid shear stress to the cells for culture, a three-dimensional tissue which has thicker tissue and in which a brain blood vessel tube network structure is more widely distributed can be produced. Furthermore, by applying fluid shear stress to the cells for culture, an excellent three-dimensional tissue more highly expressing transporters and tight junctions can be produced as a brain blood vessel model.

Means for generating a shear stress in shear stress culture can be appropriately selected based on the knowledge in the technical field. For example, culture can be performed in a microfluidic device or other devices generating shear stress. In addition, the conditions for shear stress culture can also be appropriately set based on the knowledge in the technical field.

### <Brain Blood Vessel Model-Forming Agent>

A brain blood vessel model-forming agent according to the present embodiment contains defibrated extracellular matrix components. The average length and average diameter of defibrated extracellular matrix components may be within the above-described ranges, respectively. The length of 95% of the total defibrated extracellular matrix components may be within the above-described ranges, or the length of 99% of the total defibrated extracellular matrix components may be within the above-described ranges. The diameter of 95% of the total defibrated extracellular matrix components may be within the above-described ranges, or the diameter of 99% of the total defibrated extracellular matrix components may be within the above-described ranges.

The "brain blood vessel model-forming agent" means a reagent for producing a brain blood vessel model. The brain blood vessel model-forming agent may be in the form of powder or a dispersion in which defibrated extracellular matrix components are dispersed in an aqueous medium. Examples of methods for forming defibrated extracellular matrix components and method for using the above-described forming agent include the same methods as those shown in the above-described method for producing a brain blood vessel model.

### <Device>

A device according to one embodiment includes: a plate in which at least one well is provided; and a brain blood vessel model which is placed in the well and formed through self-organization.

In the device according to the present embodiment, a brain blood vessel tube network is reproduced in a well. Therefore, the device can be suitably used as an evaluation device for examining an influence of a subject on blood-brain barrier (BBB) function.

"Self-organization" is a phenomenon in which tissue is naturally formed by interactions of constituent elements themselves. A brain blood vessel model formed through self-organization is not particularly limited, but can be formed, for example, by culturing defibrated extracellular matrix components, cells including brain microvascular endothelial cells, pericytes, and astrocytes, and as necessary, the above-described other components. A brain blood vessel model formed through self-organization may be composed of, for example, a three-dimensional tissue which contains defibrated extracellular matrix components and cells including brain microvascular endothelial cells, pericytes, and astrocytes, wherein at least a portion of the cells adheres to the defibrated extracellular matrix components.

The well may have a shape, a volume, a material, and the like so as to place a brain blood vessel model formed through self-organization therein. Examples of shapes of the well include a flat-bottom recess, a round-bottom recess, a U-bottom recess, and a V-bottom recess. The number of wells may be, for example, greater than or equal to 2, greater than or equal to 5, or greater than or equal to 50, and may be less than or equal to 100. As a plate in which at least one well is provided, a multi-well plate can be used, for example. A commercially available product can be used as a multi-well plate. For example, a multi-well plate with 6 wells, 12 wells, 24 wells, 48 wells, or 96 wells can be used. The material of the wells or the plate is not particularly limited, and can be appropriately selected depending on the purpose. Examples thereof include polystyrene, polypropylene, polyethylene, fluororesins, acrylic resins, polycarbonates, polyurethanes, and polyvinyl chloride, polyethylene terephthalate. Regarding the color of the wells and the plate, the wells and the plate may be, for example, transparent, translucent, colored, or completely shaded.

A brain blood vessel model formed through self-organization can be placed in at least a part in a flow path of a microfluidic chip. For example, a housing chamber for housing a brain blood vessel model may be provided in at least a part in a flow path of a microfluidic chip, and the brain blood vessel model may be placed in the housing chamber When a brain blood vessel model is placed in at least a part in a flow path of a microfluidic chip, perfusion culture and transfer of drugs to the periphery can be evaluated.

In addition, another biological tissue model (for example, a small intestine model, a liver model, or a kidney model) can be placed in the same flow path (for example, on an upstream side of the brain blood vessel model) as that of the microfluidic chip in which the brain blood vessel model is placed. A plurality of other biological tissue models may be placed in the same flow path as that in which the brain blood vessel model is placed. The other biological tissue models may be placed in a housing chamber in the flow path provided for housing biological tissue models. For example, a small intestine model, a liver model or a kidney model, and a brain blood vessel model may be arranged in the flow path of the microfluidic chip in this order from the upstream side. For example, a small intestine model, a liver model and/or a kidney model, and a brain blood vessel model may be arranged in the flow path of the microfluidic chip in this order from the upstream side. When the brain blood vessel model is placed in the same flow path as that of other biological tissue models, it is possible to evaluate blood-brain barrier dysfunction, cerebral transferability, and central toxicity expression of metabolites of each biological tissue.

### [Examples]

Hereinafter, the present invention will be described in more detail based on examples. However, the present invention is not limited to the following examples.

### <Test Example 1: Production of Defibrated Collagen (CMF) Using Type I Collagen>

50 mg of a porcine skin-derived type I collagen freeze-dried substance manufactured by NH Foods Ltd. was suspended in 5 mL of ultrapure water, and then homogenized with a homogenizer for 2 minutes. As a result, a dispersion containing defibrated collagen (CMF) was obtained. The dispersion containing CMF was freeze-dried by FDU-2200 (manufactured by Tokyo Rikakikai Co., Ltd.) for 3 days to remove moisture. As a result, CMF was obtained as a dried product. The diameter of the obtained CMF was 20 to 30 µm, and the average length (length) thereof was 100 to 200 µm. The diameter and the length of CMF was obtained by analyzing individual CMF components using an electron microscope.

### <Test Example 2: Production 1 of Three-Dimensional Tissue >

CMF was dispersed in a medium (DMEM) containing serum so as to have a concentration of 10 mg/mL, and a medium dispersion containing CMF was prepared.

The medium dispersion containing CMF, cells containing ciBMEC, ciBPC, and ciASTR as human established cell lines, and a medium were added to a Petri dish to bring these components into contact with each other. The cells in the obtained mixed liquid were cultured through precipitation culture for 7 days.

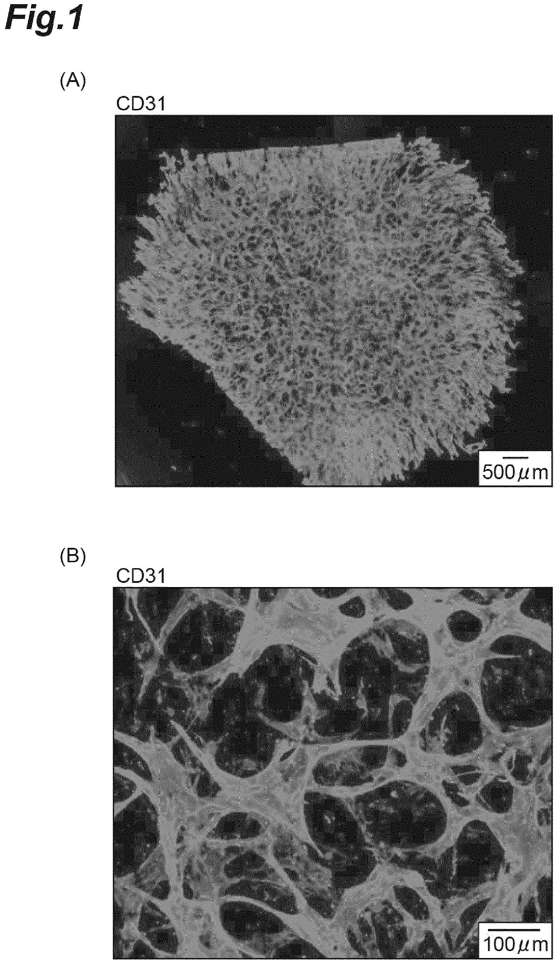

A three-dimensional tissue obtained after the culture was fluorescently labeled through fluorescent immunostaining using an anti-CD31 antibody (manufactured by DAKO, M0823) and an Alexa 647-labeled secondary antibody (manufactured by Invitrogen, A-21235). The fluorescently labeled three-dimensional tissue was observed with Confocal Quantitative Image Cytometer CQ1 (manufactured by Yokogawa Electric Corporation). The results thereof are shown in Fig. 1.

As shown in Fig. 1, it was confirmed that a brain blood vessel tube network was formed by the formation of the three-dimensional tissue using CMF.

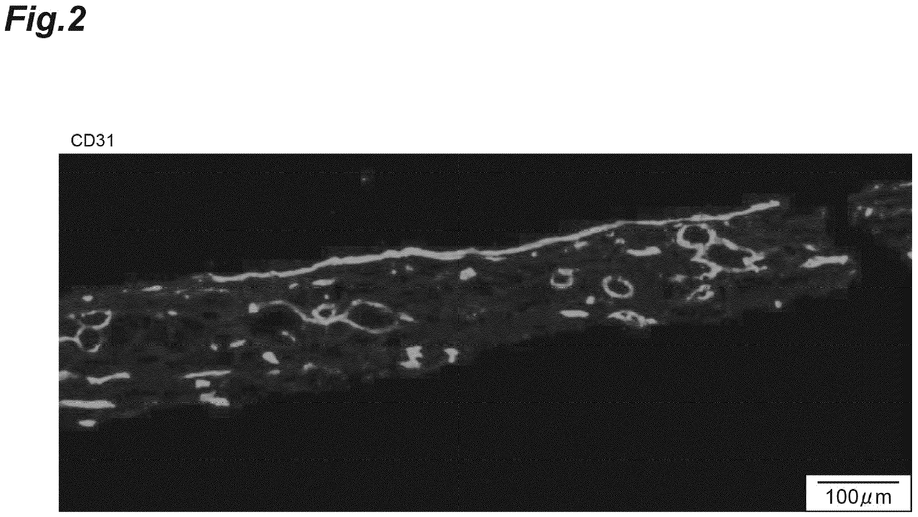

Fig. 2 is a fluorescent immunostaining photograph illustrating a cut surface of a frozen section of the produced three-dimensional tissue. Immunostaining was performed through CD31 staining. As shown in Fig. 2, the three-dimensional tissue produced using CMF had holes. It was confirmed that the brain blood vessel model composed of the three-dimensional tissue produced using CMF had a tube structure.

### <Test Example 3: Confirmation 1 of BBB Protein Expression>

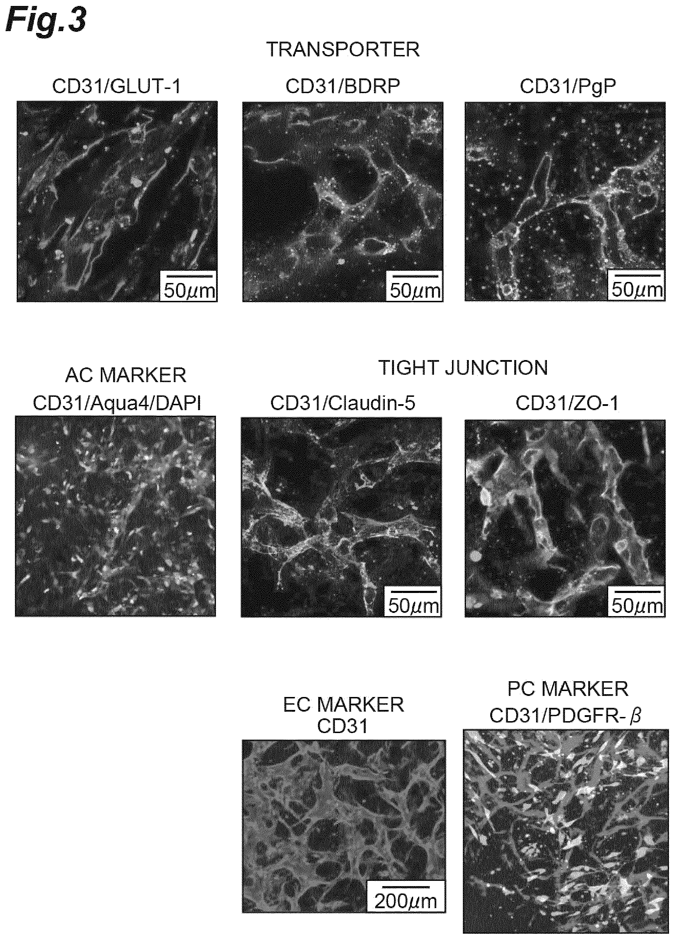

Proteins expressed in the three-dimensional tissue produced through the method described in Test Example 2 were confirmed through immunostaining. This test was carried out under the following conditions. The results are shown in Fig. 3. The three-dimensional tissue was immersed in various primary antibody solutions overnight and immersed in PBS several times for washing. Thereafter, the three-dimensional tissue was immersed in various secondary antibodies for several hours and washed with PBS.

As shown in Fig. 3, expression of transporters, tight junctions, and cell markers in the three-dimensional tissue produced using CMF was confirmed through immunostaining.

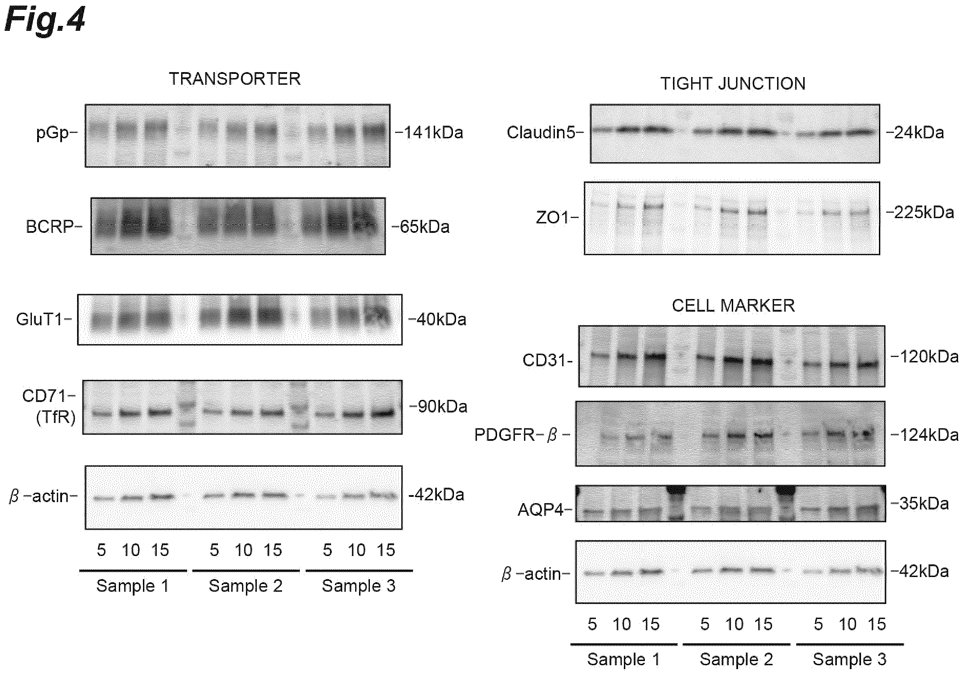

Proteins expressed in the three-dimensional tissue produced through the method described in Test Example 2 were confirmed through western blotting (WB). This test was carried out under the following conditions. The results are shown in Fig. 4. The specimen was dissolved in Extraction Buffer 5X PTR (ab1939720) to measure the concentration of proteins. Thereafter, the dissolved specimen was dispersed using 2X Sample Buffer (62.5 mM Tris, 2% SDS, 10% glycerin, 0.0125% bromophenol blue, pH 6.8). The obtained sample was separated by SDS-PAGE and transferred to a PVDF membrane. After the membrane was blocked overnight at 4°C, the membrane was reacted with a target protein-specific primary antibody and washed, and was then reacted with an HRP-labeled secondary antibody to detect a band of a target protein through a chemiluminescence method.

As shown in Fig. 4, expression of transporters, tight junctions, and cell markers in the three-dimensional tissue produced using CMF was confirmed through western blotting.

The basic performance of the brain blood vessel network was confirmed with a protein expression level by the immunostaining and western blotting.

### <Test Example 4: Production 2 of Three-Dimensional Tissue >

CMF was dispersed in a medium (DMEM) containing serum so as to have a concentration of 10 mg/mL, and a medium dispersion containing CMF was prepared.

The medium dispersion containing CMF, cells containing ciBMEC, ciBPC, and ciASTR derived from iPS cells, and a medium were added to a Petri dish to bring these components into contact with each other. The cells in the obtained mixed liquid were cultured through precipitation culture for 7 days.

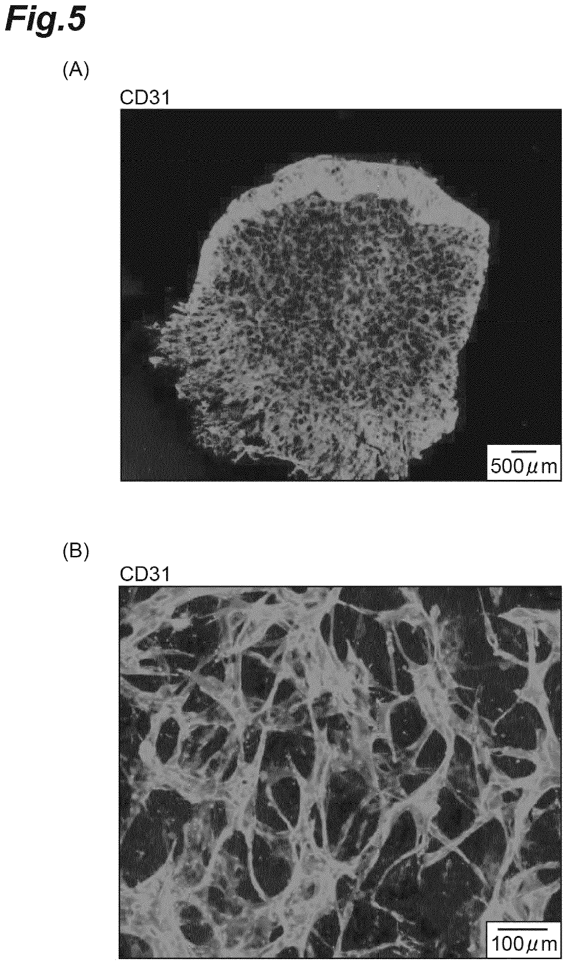

A three-dimensional tissue obtained after the culture was fluorescently labeled through fluorescent immunostaining using an anti-CD31 antibody (manufactured by DAKO, M0823) and an Alexa 647-labeled secondary antibody (manufactured by Invitrogen, A-21235). The fluorescently labeled three-dimensional tissue was observed with Confocal Quantitative Image Cytometer CQ1 (manufactured by Yokogawa Electric Corporation). The results thereof are shown in Fig. 5.

As shown in Fig. 5, it was confirmed that a brain blood vessel tube network was formed by the formation of the three-dimensional tissue using CMF even in a case where iBMEC derived from iPS cells was used.

### <Test Example 5: Production of Brain blood vessel Opening Model>

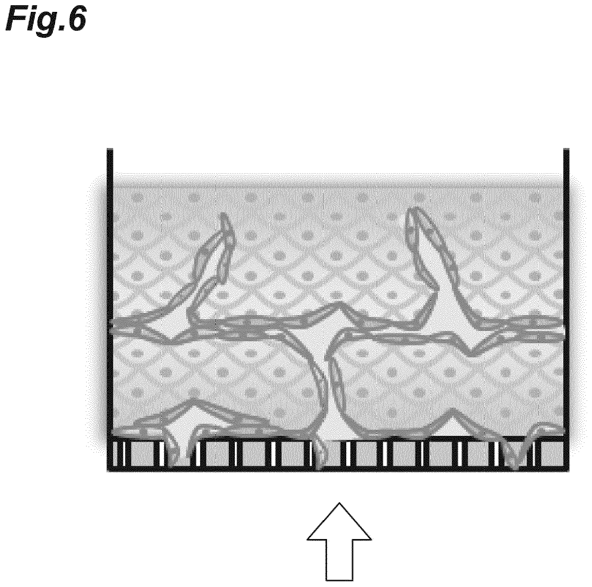

Fig. 6 is a schematic diagram illustrating one aspect of a brain blood vessel model. The brain blood vessel model shown in Fig. 6 has openings in its lower portion. The brain blood vessel model having openings in its lower portion as shown in Fig. 6 was produced through the following method. Cerebrovascular endothelial cells were adhered to a cell culture insert to produce a three-dimensional tissue thereon through the same method as that described above using CMF and cultured for several days.

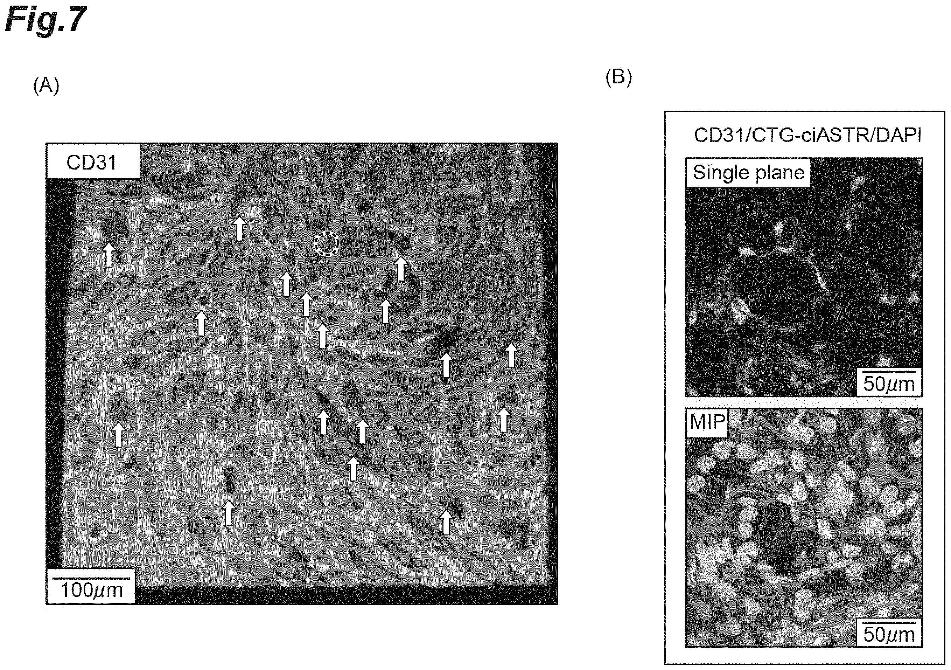

A Result obtained by observing the produced brain blood vessel model from a side having openings (the direction indicated by the arrow in Fig. 6) is shown in Fig. 7(A). Fig. 7(B) shows a region within the broken line in Fig. 7(A). The produced brain blood vessel model had a plurality of openings in its lower portion as shown by the arrows in Fig. 7(A).

### <Test Example 6: Confirmation of Opening Structure of Brain Blood Vessel Model due to Addition of FD-2000 kDa>

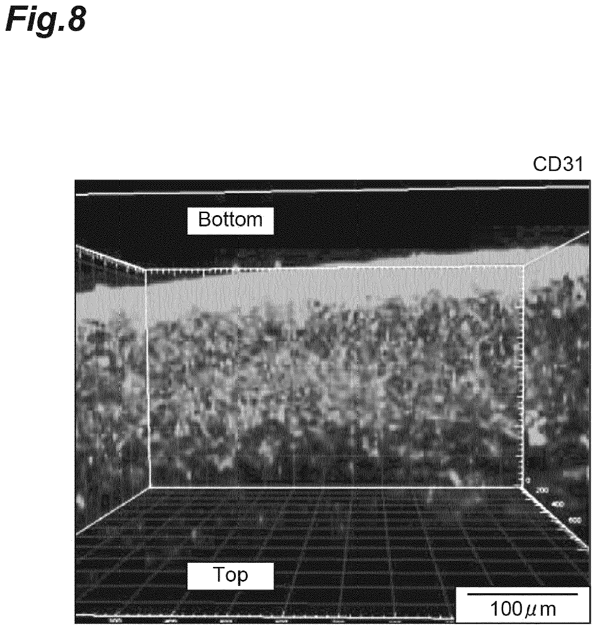

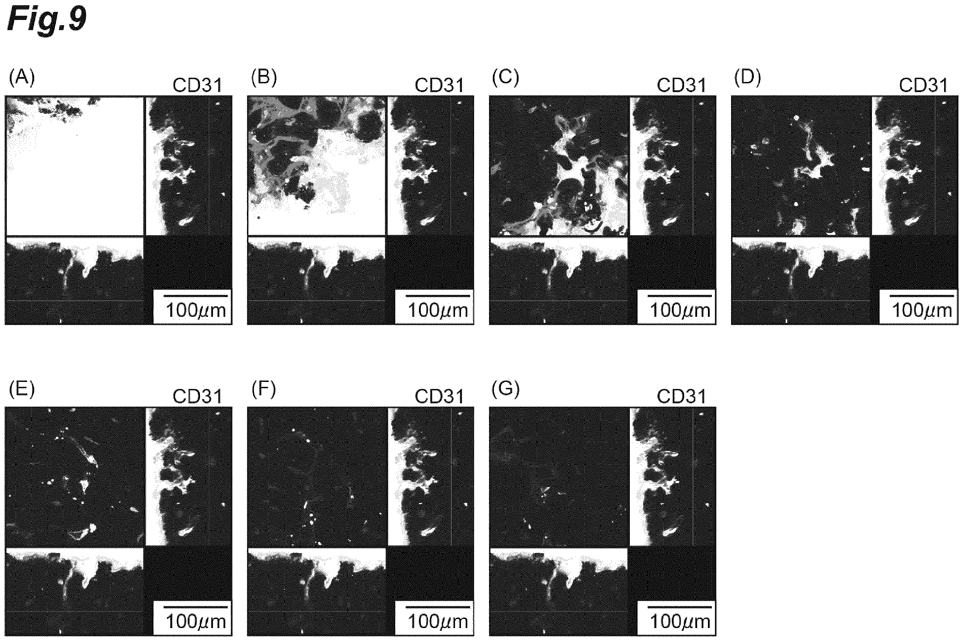

An opening structure of a brain blood vessel model due to addition of fluorescein isothiocyanate-labeled dextran FITC-Dex2000k (about 30 nm, FD-2000 kDa) (manufactured by Tokyo Chemical Industry Co., Ltd.) was confirmed. As the brain blood vessel model, one produced through the method described in Test Example 5 was used The results are shown in Figs. 8 and 9. FD-2000 kDa dissolved in a phenol red-free medium was added to outside of a cell culture insert in which the brain blood vessel model immunostained with an anti-CD31 antibody was placed. The resultant was observed using a confocal laser microscope after about 9 hours. Fig. 8 shows observation results of the brain blood vessel model having openings in its lower portion through CD31 staining.

Fig. 9 shows observation results of behavior of fluorescein isothiocyanate-labeled dextran (FD-2000 kDa) inside openings of the CD31-positive brain blood vessel model. The inside of square frames in Figs. 9(A) to 9(G) shows transverse sections of the three-dimensional tissue located vertically upward at 15.6 µm (Fig. 9(B)), 31.3 µm (Fig. 9(C)), 46.9 µm (Fig. 9 (D)), 62.5 µm (Fig. 9(E)), 93.8 µm (Fig. 9(F)), or 125 µm (Fig. 9(G)) with the bottom surface (the lower surface of the three-dimensional tissue) of a well of the cell culture insert, in which the three-dimensional tissue is formed, as a reference (0 µm, Fig. 9(B)). The white regions in Fig. 9 indicate that there is fluorescein isothiocyanate-labeled dextran (FD-2000 kDa), and the lightly colored regions in Fig. 9 indicate CD31-positive regions.

As shown in Figs. 8 and 9, it was suggested that fluorescein isothiocyanate-labeled dextran (FD-2000 kDa) was contained in the openings of the CD31-positive brain blood vessel model.

### <Test Example 7: Analysis of Defibrated Collagen Component (CMF)>

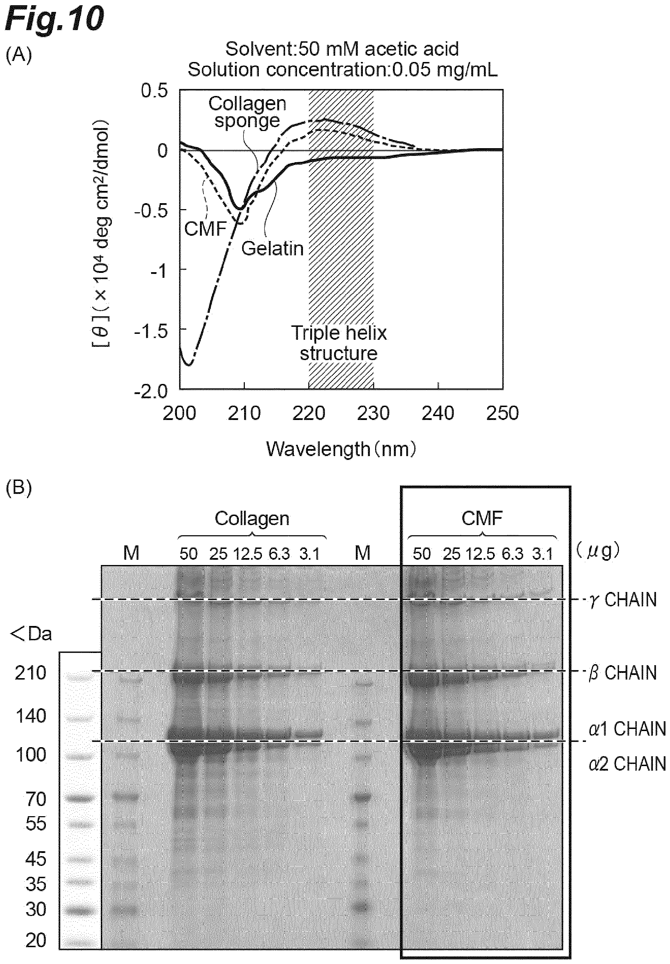

A defibrated collagen component (CMF) was analyzed using a circular dichroism spectrum and SDS-PAGE. The results are shown in Table 10. As shown in Fig. 10, it was confirmed that a triple helix structure and a molecular weight were maintained in the defibrated collagen component (CMF).

### <Test Example 8: Production 3 of Three-Dimensional Tissue > (Shear Stress Culture)

0.7 mg of CMF produced through the same method as that in Test Example 1, 0.4 mg of fibrinogen, and 0.3 U of thrombin were mixed with 2 × 10⁵ human cerebrovascular endothelial cells (HBEC), 4 × 10⁵ human astrocytes (HA), and 1 × 10⁵ human pericytes (HP) to obtain a mixed solution. 70 µL of the mixed solution was added to 24-well insert and set in one culture chamber of a pressure-driven type micro-flow path device (refer to Republished Japanese Translation No. 2016/158233 of the PCT International Publication for Patent Applications) including a unit of an articulated culture container in which two culture chambers communicate with each other through a plurality of communication flow paths. Thereafter, 1.4 mL of a liquid medium was added to the same culture chamber, and culture was carried out under the conditions of alternately pressurizing the two culture chambers to 10 kPa at intervals of 30 seconds to 60 seconds for7 days to produce a three-dimensional tissue. The above-described micro-flow path device has a mechanism in which the liquid medium is fed between the two culture chambers due to a pressure difference caused by pressurization (refer to Figs. 9D and 9E in Republished Japanese Translation No. 2016/158233 of the PCT International Publication for Patent Applications). After the second day of culture, the medium was changed every day.

### (Static Culture)

70 µL of the above-described mixed solution was added to a Petri dish, and culture was carried out for 7 days after gelation through incubation for 1 hour to produce a three-dimensional tissue as a comparative example.

The three-dimensional tissue obtained after the culture were fluorescently labeled through fluorescent immunostaining using an anti-CD31 antibody (manufactured by DAKO, M0823) and an Alexa 647-labeled secondary antibody (manufactured by Invitrogen, A-21235). The fluorescently labeled three-dimensional tissue were observed with Confocal Quantitative Image Cytometer CQ1 (manufactured by Yokogawa Electric Corporation). The results are shown in Table 11.

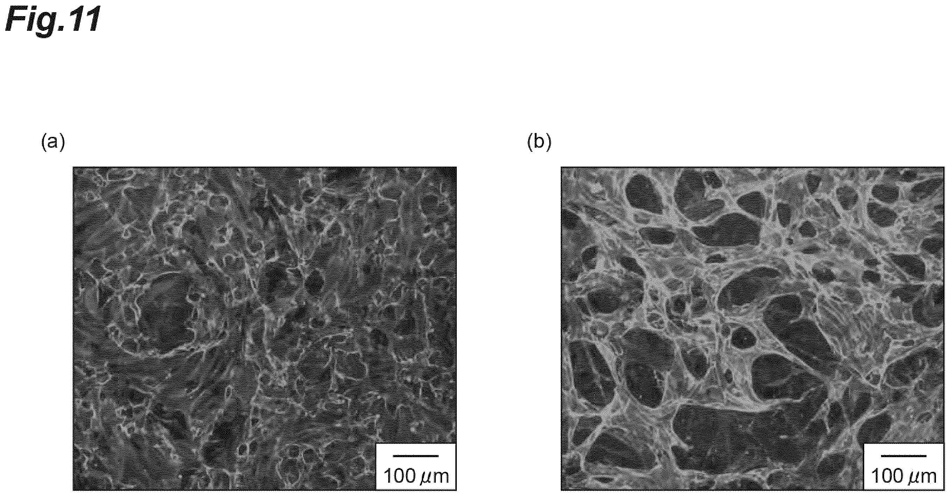

As shown in Fig. 11, it was confirmed that a clearer brain blood vessel tube network structure was formed in (b) the three-dimensional tissue produced by shear stress culture compared with (a) the three-dimensional tissue produced by static culture.

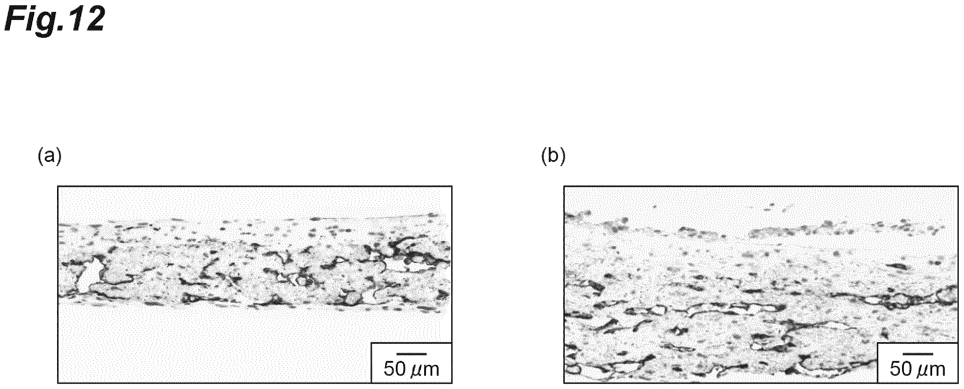

In addition, the three-dimensional tissue obtained after the culture were subjected to DAB staining using an anti-CD31 antibody (manufactured by DAKO, M0823). Cell nuclei were stained with toluidine blue. Results obtained by observing the stained three-dimensional tissue with EVOS FL Auto 2 Cell Imaging System (manufactured by Thermo Fisher Scientific Inc.) are shown in Fig. 12.

As shown in Fig. 12, although clear lumens were observed in both three-dimensional tissue, it was confirmed that a brain blood vessel tube network structure was widely distributed in (b) the three-dimensional tissue produced by shear stress culture compared with (a) the three-dimensional tissue produced by static culture.

### <Test Example 9: BBB Protein Gene Expression Analysis>

In addition, the three-dimensional tissue obtained after the culture were subjected to DNA extraction and real-time PCR to analyze gene expression of transporters and tight junctions. The DNA extraction was carried out using R2081 manufactured by Zymo Research through the method as described in its manual. The real-time PCR was carried out using a kit (THUNDERBIRDR Probe qPCR RTSet (manufactured by TOYOBO) through the method as described in its manual.

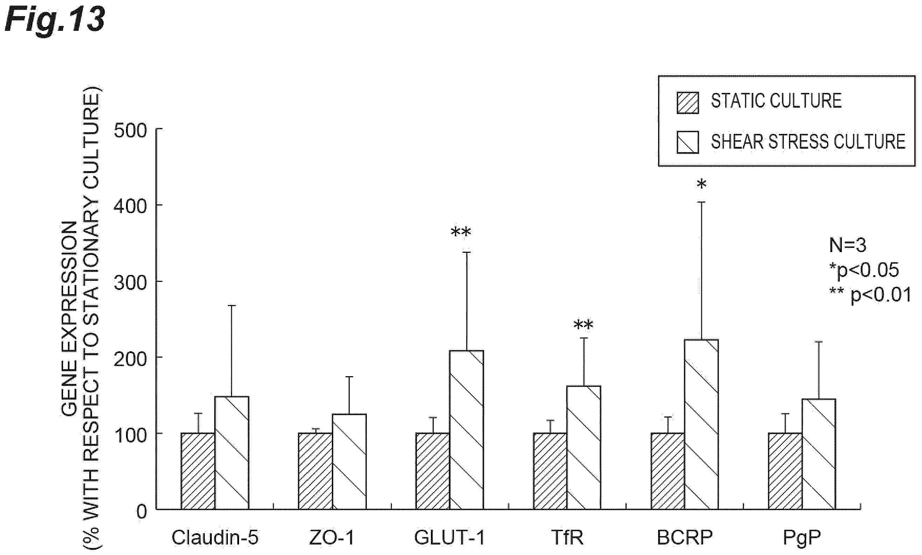

Standardization was performed with the expression level of the CD31 gene, expression levels of genes in the three-dimensional tissue produced by static culture were set to 100%, and expression levels of the genes in the three-dimensional tissue produced by shear stress culture were compared therewith by ratios. The results are shown in Fig. 13. The expression levels of the genes in the three-dimensional tissue produced by shear stress culture tended to increase in both transporter and tight junction proteins.

### <Test Example 10: Confirmation 2 of BBB Protein Expression>

In addition, proteins expressed in the three-dimensional tissue obtained after the culture were confirmed through fluorescent immunostaining. The fluorescent immunostaining using various primary antibodies and labeled secondary antibodies corresponding thereto was performed to fluorescently label the three-dimensional tissue. The fluorescently labeled three-dimensional tissue were observed with Confocal Quantitative Image Cytometer CQ1 (manufactured by Yokogawa Electric Corporation).

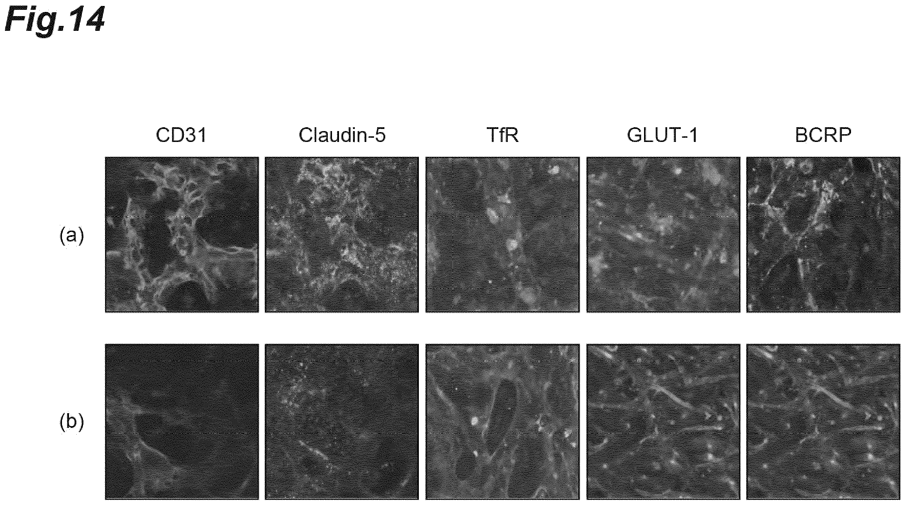

As shown in Fig. 14, expression of proteins of transporters, tight junctions, and vascular endothelial cell markers was confirmed in both of (b) the three-dimensional tissue produced by shear stress culture and (a) the three-dimensional tissue produced through a static culture.

## Claims

1. A brain blood vessel model composed of a three-dimensional tissue comprising defibrated extracellular matrix components and cells including brain microvascular endothelial cells, pericytes, and astrocytes,
wherein at least a portion of the cells adheres to the defibrated extracellular matrix components.

2. The brain blood vessel model according to claim 1,
wherein the defibrated extracellular matrix components are defibrated collagen components.

3. The brain blood vessel model according to claim 1 or 2,
wherein the three-dimensional tissue further comprises fibrin.

4. A method for producing a brain blood vessel model, comprising:
a contacting step of bringing defibrated extracellular matrix components into contact with cells including brain microvascular endothelial cells, pericytes, and astrocytes in an aqueous medium; and
a culture step of culturing the cells with which the defibrated extracellular matrix components are brought into contact.

5. The method for producing a brain blood vessel model according to claim 4,
wherein the defibrated extracellular matrix components are defibrated collagen components.

6. The method for producing a brain blood vessel model according to claim 4 or 5,
wherein the contacting step is performed in the presence of fibrin.

7. A device comprising:
a plate in which at least one well is provided; and
a brain blood vessel model which is placed in the well and formed through self-organization.

8. The device according to claim 7,
wherein the brain blood vessel model is composed of a three-dimensional tissue comprising defibrated extracellular matrix components and cells including brain microvascular endothelial cells, pericytes, and astrocytes,
wherein at least a portion of the cells adheres to the defibrated extracellular matrix components.
